# EUROPEAN PATENT APPLICATION

(11) **EP 4 740 950 A1**
(43) Date of publication of application: **13.05.2026**
(21) Application number: 24836031.5
(22) Date of filing: 02.07.2024
(51) Int. Cl.: A61K 31/685, A61P 1/16, A61P 3/10, A61P 9/04, A61P 11/00, A61P 13/12, A61P 21/00, A61P 29/00, A61P 31/00, A61P 31/04, A61P 35/00, A61P 37/06, C12N 15/09, C12Q 1/06, G01N 33/15, G01N 33/50

(54) **PROPHYLACTIC OR AMELIORATING AGENT FOR CACHEXIA**

(30) Priority: 04.07.2023 JP 2023109812
(71) Applicant: The University of Osaka, Osaka 565-0871 (JP); Kewpie Corporation, Tokyo 150-0002 (JP)
(72) Inventor: TAKAKURA Nobuyuki, Suita-shi, Osaka 565-0871 (JP); RAHMAWATI Fitriana Nur, Suita-shi, Osaka 565-0871 (JP); UCHIDA Tomonori, Chofu-shi, Tokyo 182-0002 (JP); HOSHINA Ryosuke, Chofu-shi, Tokyo 182-0002 (JP)
(74) Representative: Vossius & Partner Patentanwälte Rechtsanwälte mbB
(86) International application number: PCT/JP2024/023897
(87) International publication number: WO 2025/009518

(57) **Abstract**

The present invention provides a preventing or ameliorating agent for cachexia, comprising a phospholipid and/or a lysophospholipid as an active ingredient; and a method for screening for a substance for ameliorating cachexia. The preventing or ameliorating agent for cachexia according to the present invention is effective for not only cancer cachexia but also cachexia caused by diseases other than cancer, inhibits skeletal muscle mass loss, inhibits lipoatrophy, and inhibits the expression of proinflammatory cytokines.

## Description

### TECHNICAL FIELD

The present invention relates to a preventing or ameliorating agent for cachexia.

### BACKGROUND ART

Cachexia is the term referring to wasting of the body due to malnutrition. The definition of cachexia was emerged in a consensus conference held by European and American experts in 2007 and is as follows: "cachexia, is a complex metabolic syndrome associated with underlying illness and characterized by loss of muscle with or without loss of fat mass. The prominent clinical feature of cachexia is weight loss in adults or growth failure in children. Anorexia, inflammation, insulin resistance and increased muscle protein breakdown are frequently associated with cachexia. Cachexia is distinct from starvation, age-related loss of muscle mass, primary depression, malabsorption and hyperthyroidism and is associated with increased morbidity" (Non-Patent Literature 1). Cachexia is especially characterized by loss of skeletal muscle mass. Wasting of skeletal muscle has a huge impact on the quality of life and the vital prognosis of the patients, and such a condition has become known as sarcopenia.

Once cachexia is developed, motor ability and other abilities are reduced, and the recovery ability of the body declines. As a result, many diseases may become refractory to treatments, and the patients may lose the opportunity to receive therapy. As well known, the transition to cachexia in cancer patients may have a huge impact on the prognosis of most of the patients by taking away the opportunity to receive cancer therapy using therapeutic cancer drugs, which usually have substantial influence or adverse side effects on normal tissue. Needless to say, cachexia is associated with not only cancer but also many chronic wasting diseases, including chronic heart failure, chronic obstructive lung disease, chronic renal failure, chronic liver diseases, chronic infection and sepsis (e.g., AIDS, etc.), autoimmune diseases such as rheumatism, and diabetes mellitus. If cachexia occurs in patients with these diseases, the patients may become refractory to treatments.

Currently, locally or systemically enhanced expression of proinflammatory cytokines has been considered a key causative factor of cachexia (Non-Patent Literature 2). However, the whole picture of cachexia has not been clarified yet. The pathology of cachexia is believed to involve a complex interplay of various factors, including metabolic disorders due to persistent tissue inflammation causing tissue damage and the acceleration of catabolism, and the loss of energy intake by lack of appetite or other causes.

The elucidation of the molecular mechanisms of cachexia at the basic medical level is required, and there is a need for the development of a therapeutic drug capable of inhibiting cachexia. Conventionally, anti-inflammatory drugs, appetite-improving drugs (ghrelin receptor agonists), or selective androgen receptor modifiers with anabolism-promoting action and other drugs are applied to patients with cachexia. However, the effects of these drugs are limited, and the emergence of a therapeutic drug that is expected to significantly ameliorate cachexia has been desired.

### CITATION LIST

### NON-PATENT LITERATURE

Non-Patent Literature 1: Evans WJ et al.: Cachexia: a new definition. Clinical Nutrition 2008; 27: 793-799.
Non-Patent Literature 2: Cederholm T et al.: ESPEN guidelines on definitions and terminology of clinical nutrition. Clinical Nutrition 2017; 36: 49-64.

### SUMMARY OF INVENTION

### TECHNICAL PROBLEM

An object of the present invention is to provide a cachexia-preventing or ameliorating agent that is effective for not only cancer cachexia but also cachexia caused by diseases other than cancer. Another object of the present invention is to provide a screening method for a substance for ameliorating cachexia.

### SOLUTION TO PROBLEM

The present invention was made to solve the above problems and includes the following.
[1] A preventing or ameliorating agent for cachexia, comprising a phospholipid and/or a lysophospholipid as an active ingredient.
[2] The preventing or ameliorating agent according to the above [1], wherein the phospholipid is a phosphatidylcholine or a phosphatidylserine.
[3] The preventing or ameliorating agent according to the above [1], wherein the lysophospholipid is a lysophosphatidylcholine or a lysophosphatidylserine.
[4] The preventing or ameliorating agent according to any one of the above [1] to [3], wherein the agent is used for inhibiting skeletal muscle mass loss.
[5] The preventing or ameliorating agent according to any one of the above [1] to [3], wherein the agent is used for inhibiting lipoatrophy.
[6] The preventing or ameliorating agent according to any one of the above [1] to [3], wherein the agent is used for inhibiting expression of a proinflammatory cytokine.
[7] The preventing or ameliorating agent according to any one of the above [1] to [6], wherein the cachexia is induced by a disease, wherein the disease that induces the cachexia is a chronic disease associated with inflammation.
[8] The preventing or ameliorating agent according to the above [7], wherein the chronic disease associated with inflammation is a disease selected from the group consisting of cancer, chronic heart failure, chronic obstructive lung disease, chronic renal failure, chronic liver diseases, chronic infections, sepsis, autoimmune diseases, and diabetes mellitus.
[9] A method for screening for a substance for ameliorating cachexia, comprising selecting a test substance that promotes myotube formation as compared to that without addition of the test substance when culture is performed in an in vitro differentiation induction system of a stem cell or a progenitor cell into a skeletal muscle cell using a differentiation induction medium containing a culture supernatant of a cancer cell with addition of the test substance.
[10] A method for screening for a substance for ameliorating cachexia, comprising selecting a test substance that promotes adipocyte differentiation as compared to that without addition of the test substance when culture is performed in an in vitro differentiation induction system of a stem cell or a progenitor cell into an adipocyte using a differentiation induction medium containing a culture supernatant of a cancer cell with addition of the test substance.

### ADVANTAGEOUS EFFECTS OF INVENTION

The present invention provides a cachexia-preventing or ameliorating agent that is effective for not only cancer cachexia but also cachexia caused by diseases other than cancer. The present invention also provides a screening method for a substance for ameliorating cachexia.

### BRIEF DESCRIPTION OF DRAWINGS

Fig. 1 shows the results of examination of the effect of phosphatidylcholine (PC) and lysophosphatidylcholine (LPC) on the inhibition of myotube formation due to a cachexic substance contained in the culture supernatant of colon cancer cells (Colon26) in an in vitro differentiation induction system of myoblasts (C2C12) into skeletal muscle cells.
Fig. 2 shows the results of examination of the effect of LPC on the inhibition of differentiation due to a cachexic substance contained in the culture supernatant of colon cancer cells (Colon26) in an in vitro differentiation induction system of mesenchymal cells (OP9) into adipocytes.
Fig. 3 shows the results of measuring the weight of the gastrocnemius muscle (Gastrocnemius) and the tibialis muscle (Tibialis) harvested on day 36 post-transplantation from tumor-bearing mice that were developed by subcutaneous transplantation of colon cancer cells (Colon26) and then received intravenous administration of PC.
Fig. 4 shows the results of measuring the expression levels of the MuRF-1 and Atrogin-1 genes, which induce the degradation of muscle, by quantitative PCR using RNA extracted on day 36 post-transplantation from the muscle of tumor-bearing mice that were developed by subcutaneous transplantation of colon cancer cells (Colon26) and then received intravenous administration of PC.
Fig. 5 shows the result of measuring the weight of the epididymal fat tissue on day 36 post-transplantation in tumor-bearing mice that were developed by subcutaneous transplantation of colon cancer cells (Colon26) and then received intravenous administration of PC.
Fig. 6 shows the result of measuring the expression levels of the Leptin gene, which has the function of maintaining fat tissue, by quantitative PCR using RNA extracted on day 36 post-transplantation from the epididymal fat tissue of tumor-bearing mice that were developed by subcutaneous transplantation of colon cancer cells (Colon26) and then received intravenous administration of PC.
Fig. 7 shows the result of calculation of the survival rate in tumor-bearing mice that were developed by subcutaneous transplantation of colon cancer cells (Colon26) and then received intravenous administration of PC.
Fig. 8 shows the results of measuring the weight of the gastrocnemius muscle (Gastrocnemius) and the tibialis muscle (Tibialis) harvested on day 24 post-transplantation from tumor-bearing mice that were developed by subcutaneous transplantation of colon cancer cells (Colon26) and then received oral administration of PC, LPC or a PC/LPC mixture.
Fig. 9 shows the hematoxylin and eosin staining of histological sections prepared from the gastrocnemius muscle that was subjected to measurement of weight in Fig. 8.
Fig. 10 shows the result of measuring the circumference of muscle fibers in 50 fibers per mouse in each group in the histological images in Fig. 9.
Fig. 11 shows the results of measuring the expression levels of the MuRF-1 and Atrogin-1 genes, which induce the degradation of muscle, by quantitative PCR using RNA extracted from the muscle tissue that was subjected to measurement of weight in Fig. 8.
Fig. 12 shows the result of measuring the serum IL-6 levels in the blood collected on day 24 post-transplantation of colon cancer cells in the mice used in the experiment shown in Fig. 8.
Fig. 13 shows the result of measuring the blood glucose levels in streptozocin (STZ)-induced type 1 diabetic model mice on days 0, 7 and 31 after oral administration of either of two types of PC/LPC mixtures starting from day 7 post-administration of STZ.
Fig. 14 shows the results of measuring the weight of the gastrocnemius muscle (Gastrocnemius) and the tibialis muscle (Tibialis) on day 31 post-administration of STZ in the experiment shown in Fig. 13.
Fig. 15 shows the results of measuring the expression levels of the MuRF-1 and Atrogin-1 genes, which induce the degradation of muscle, by quantitative PCR using RNA extracted from the muscle tissue that was subjected to measurement of weight in Fig. 14.
Fig. 16 shows the appearance of the epididymal fat tissue observed by making an incision in the abdomen of mice under anesthesia on day 24 post-transplantation in tumor-bearing mice that were developed by subcutaneous transplantation of colon cancer cells (Colon26) and then received oral administration of PC, LPC or a PC/LPC mixture.
Fig. 17 shows the result of measuring the weight of the epididymal fat tissue harvested after the observation of the appearance in Fig. 16.
Fig. 18 shows the hematoxylin and eosin staining of histological sections prepared from the epididymal fat tissue used to measure the weight in Fig. 17.
Fig. 19 shows the result of measuring the circumference of adipocytes in 50 cells per mouse in each group in the histological images in Fig. 18.
Fig. 20 shows the result of measuring the serum oncostatin M (OMS) levels in the blood collected on day 24 post-transplantation of colon cancer cells in the mice used in the experiment shown in Fig. 16.
Fig. 21 shows the result of measuring the IL-6 levels in the culture supernatant of colon cancer cells (Colon26) treated with PC or phosphatidylserine (PS).

### DESCRIPTION OF EMBODIMENTS

### Preventing or ameliorating agent for cachexia

The present invention provides a preventing or ameliorating agent for cachexia, comprising a phospholipid and/or a lysophospholipid as an active ingredient (hereinafter referred to as the "ameliorating agent of the present invention"). The ameliorating agent of the present invention may comprise a phospholipid only, a lysophospholipid only, or both a phospholipid and a lysophospholipid.

The phospholipid used in the ameliorating agent of the present invention may be a diacyl glycerophospholipid or a sphingophospholipid. The diacyl glycerophospholipid may be, for example, a phosphatidic acid, a phosphatidylcholine, a phosphatidylethanolamine, a phosphatidylserine, a phosphatidylglycerol, a phosphatidylinositol, etc. The sphingophospholipid may be, for example, a sphingomyelin, etc. The phospholipid may be a single type or a combination of two or more types. The phospholipid may be blended in the form of a composition containing a phospholipid as a major component. Examples of such a composition containing a phospholipid as a major component include PC-98N (Kewpie Corporation, phosphatidylcholine content: about 95%), etc. The phospholipid may be an extract extracted from a naturally occurring source or a chemically synthesized product. Such a phospholipid extracted from a naturally occurring source can be obtained from, for example, an animal or plant material, such as egg yolk, soybeans, or rapeseeds. The phospholipid may be hydrogenated, if necessary.

The phospholipid used in the ameliorating agent of the present invention is not particularly limited, but is preferably a phosphatidylcholine (PC). The phosphatidylcholine (also referred to as lecithin) may be any phosphatidylcholine having a structure in which fatty acids are attached to the C1 and C2 positions of glycerol, respectively, and phosphocholine is attached to the C3 position of the glycerol. Examples of the phosphatidylcholine include egg yolk lecithin, soybean lecithin, soybean phosphatidylcholine, dioctanoylphosphatidylcholine, dinonanoylphosphatidylcholine, didecanoylphosphatidylcholine, diundecanoylphosphatidylcholine, dilauroylphosphatidylcholine, dimyristoylphosphatidylcholine, dipalmitoylphosphatidylcholine, distearoylphosphatidylcholine, dipalmitoleoylphosphatidylcholine, dioleoylphosphatidylcholine, dilinoleoylphosphatidylcholine, dieicosapentaenoylphosphatidylcholine, didocosahexaenoylphosphatidylcholine, dierucoylphosphatidylcholine, (1-myristoyl-2-palmitoyl)phosphatidylcholine, (1-palmitoyl-2-myristoyl)phosphatidylcholine, (1-oleoyl-2-palmitoyl)phosphatidylcholine, (1-palmitoyl-2-oleoyl)phosphatidylcholine, etc.

The phospholipid used in the ameliorating agent of the present invention is preferably a phosphatidylserine (PS). The phosphatidylserine may be any phosphatidylserine having a structure in which fatty acids are attached to the C1 and C2 positions of glycerol, respectively, and phosphoserine is attached to the C3 position of the glycerol. Examples of the phosphatidylcholine include dioctanoylphosphatidylserine, dinonanoylphosphatidylserine, didecanoylphosphatidylserine, diundecanoylphosphatidylserine, dilauroylphosphatidylserine, dimyristoylphosphatidylserine, dipalmitoylphosphatidylserine, distearoylphosphatidylserine, dipalmitoleoylphosphatidylserine, dioleoylphosphatidylserine, dilinoleoylphosphatidylserine, dieicosapentaenoylphosphatidylserine, didocosahexaenoylphosphatidylserine, dierucoylphosphatidylserine, (1-myristoyl-2-palmitoyl)phosphatidylserine, (1-palmitoyl-2-myristoyl)phosphatidylserine, (1-oleoyl-2-palmitoyl)phosphatidylserine, (1-palmitoyl-2-oleoyl)phosphatidylserine, etc.

A lysophospholipid is a phospholipid in which one of the fatty acids is removed from the above phospholipid. The lysophospholipid used in the ameliorating agent of the present invention may be a monoacylglycerophospholipid resulting from the removal of any one of the fatty acids attached via an ester bond to the hydroxy groups of the glycerol backbone of a diacylglycerophospholipid, or a lysosphingophospholipid resulting from the removal of any one of the fatty acids attached via an amide bond to the amino group of a sphingosine backbone. Examples of the monoacylglycerophospholipid include lysophosphatidic acid, lysophosphatidylcholine, lysophosphatidylethanolamine, lysophosphatidylserine, lysophosphatidylglycerol, lysophosphatidylinositol, etc. Examples of the lysosphingophospholipid include lysosphingomyelin, sphingosine-1-phosphate, etc. The lysophospholipid may be a single type or a combination of two or more types. The lysophospholipid may be blended in the form of a composition containing a lysophospholipid as a major component. Examples of such a composition containing a lysophospholipid as a major component include LPC-1 (Kewpie Corporation, lysophosphatidylcholine content: about 95%), etc. The lysophospholipid can be obtained by treatment of the above phospholipid with an enzyme such as phospholipase or ceramide deacylase. Alternatively, the lysophospholipid can be obtained by direct treatment of an animal or plant material with an enzyme, such as phospholipase or ceramide deacylase, followed by extraction with a solvent, such as ethanol. The lysophospholipid may be hydrogenated, if necessary.

The lysophospholipid used in the ameliorating agent of the present invention is not particularly limited, but is preferably a lysophosphatidylcholine (LPC) or a lysophosphatidylserine (LPS). The lysophosphatidylcholine may be any compound resulting from the removal of one of the fatty acids from the various types of phosphatidylcholines exemplified in the above paragraph. The lysophosphatidylserine may be any compound resulting from the removal of one of the fatty acids from the various types of phosphatidylserines exemplified in the above paragraph.

The prevention of cachexia herein means the prevention of exacerbation of the condition of the subject who shows the signs of cachexia. The amelioration of cachexia means the improvement of the condition of the subject who has already developed cachexia. The symptoms of cachexia include skeletal muscle mass loss, lipoatrophy, weight loss, growth disorder, lack of appetite, anemia, edema, inflammation, exhaustion, general debility, fatigue, pain, nausea, vomiting, depression, anxiety, taste alteration, smell alteration, etc. The ameliorating agent of the present invention has been demonstrated to inhibit skeletal muscle mass loss due to cachexia, inhibit lipoatrophy due to cachexia, and inhibit the expression of proinflammatory cytokines (e.g., IL-6, oncostatin M, etc.) due to cachexia (see Examples).

Cachexia may be induced by a disease, and examples of the disease that induces cachexia include, but are not limited to, cancer, heart diseases, vascular diseases, renal diseases, liver diseases, lung diseases, intestinal diseases, urologic diseases, infections, immunological diseases, diabetes mellitus, central nervous system diseases such as Parkinson's disease, amyloidosis, psychiatric disorders, endocrine diseases, etc. The disease that induces cachexia may be a chronic disease associated with inflammation. Examples of the chronic disease associated with inflammation include cancer, chronic heart failure, chronic obstructive lung disease, chronic renal failure, chronic liver diseases, chronic infections, sepsis, autoimmune diseases, diabetes mellitus, etc.

The ameliorating agent of the present invention can be embodied as a medicament. When the ameliorating agent of the present invention is embodied as a medicament, the medicament can be formulated by appropriately combining a phospholipid and/or a lysophospholipid as an active ingredient with a pharmaceutically acceptable carrier or additive in accordance with a known production method for pharmaceutical formulations (e.g., the methods described in the Japanese pharmacopoeia, etc.). Specific examples of such a pharmaceutical formulation include oral or parenteral formulations, such as tablets (including sugar-coated tablets, film-coated tablets, sublingual tablets, orally disintegrating tablets, buccal tablets, etc.), pills, powders, granules, capsules (including soft capsules and microcapsules), troches, syrups, solutions, emulsions, suspensions, controlled release formulations (e.g., fast release formulations, sustained release formulations, sustained release microcapsules, etc.), aerosols, films (e.g., orally disintegrating films, oral mucosal adhesive films, etc.), injections (e.g., subcutaneous injection, intravenous injection, intramuscular injection, intraperitoneal injection, etc.), intravenous infusions, transdermal formulations, ointments, lotions, patches, suppositories (e.g., rectal suppository, vaginal suppository, etc.), pellets, transnasal formulations, gastric formulations, enteral formulations, transpulmonary formulations (inhalants), eye drops, etc. The blending ratio of a carrier or additive may determined as appropriate based on the amount usually used in the pharmaceutical field. The carrier or additive that can be blended is not limited to a particular one, and examples thereof include various types of carriers, such as water, physiological saline, other aqueous solvents, or aqueous or oily bases; and various types of additives, such as excipients, binders, pH adjusting agents, disintegrants, absorption enhancers, lubricants, colorants, flavor improvers or fragrances.

Additives that can be blended into tablets, capsules, etc., may include, for example, binders such as gelatin, corn starch, tragacanth, or gum arabic; excipients such as crystalline cellulose; swelling agents such as corn starch, gelatin, or alginic acid; lubricants such as magnesium stearate; sweeteners such as sucrose, lactose, or saccharin; flavoring agents such as peppermint, wintergreen oil, or cherry; etc. When the formulation unit is a capsule, a liquid carrier such as fats and oils can be further blended into the formulation, in addition to the ingredients of the above types. A sterile composition for injection can be prepared according to conventional formulation procedures (for example, by dissolving or suspending the active ingredient in a solvent, such as water for injection or a natural vegetable oil). An aqueous liquid for injection may be, for example, physiological saline or an isotonic solution containing glucose or other auxiliary agents (e.g., D-sorbitol, D-mannitol, sodium chloride, etc.). The aqueous liquid for injection may further contain an appropriate solubilizing agent, including, for example, alcohols (ethanol, etc.), polyalcohols (propylene glycol, polyethylene glycol, etc.), and nonionic surfactants (polysorbate 80, HCO-50, etc.). An oily liquid for injection may be, for example, sesame oil, soybean oil, or the like, and may further contain a solubilizing agent such as benzyl benzoate or benzyl alcohol. Additionally, the liquid for injection may further contain a buffering agent (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc.

The ameliorating agent of the present invention can be embodied as a food or drink. The food or drink includes nutritional supplement foods or drinks, health foods or drinks, functional foods or drinks, foods or drinks for sick people, foods or drinks for small children, foods or drinks for elderly people, etc. The food or drink may be in any form, including, for example, supplements, drinks, processed foods, confectionery, dairy products, fats and oil processed foods, etc. Examples of the supplements include fine granules, tablets, granules, powders, capsules (including soft capsules and hard capsules), chewables, syrups, energy drinks, natural liquid foods, low residue diets, elemental diets, gastric feeding formulations, enteral feeding formulations, etc. Examples of the drinks include tea drinks, refreshing drinks, carbonated drinks, nutritional drinks, fruit drinks, lactic drinks, etc. Examples of the processed foods include bread, noodles, pasta, ham, bacon, sausage, fish cakes, etc. Examples of the confectionery include candies, jam, chewing gum, ice cream, snack confectionery, cookies, biscuits, cakes, wafers, pastry, chocolate, Japanese traditional sweets, etc. Examples of the dairy products include processed milk, fermented milk, butter, cheese, yogurt, etc. Examples of the fat and oil processed foods include margarine, mayonnaise, shortening, whipped cream, dressing, etc.

The ameliorating agent of the present invention can be embodied as a formulation composed of a phospholipid and/or a lysophospholipid included in the cavity of a cyclic oligosaccharide. The cyclic oligosaccharide may be, for example, a cyclodextrin, etc. The ameliorating agent of the present invention can also be embodied as a self-emulsifying emulsion formulation. A self-emulsifying emulsion contains an oil component, a polyhydric alcohol, water and an emulsifier, and can form a nano-scale oil-in-water emulsion upon contact with a body fluid such as blood or a digestive juice. When the agent is in the form of a self-emulsifying emulsion, the bioavailability of the phospholipid and/or the lysophospholipid improves, and the effect of the present invention is more easily exhibited, and thus a self-emulsifying emulsion is suitable for the present invention. The self-emulsifying emulsion can be prepared by a conventional method. For example, the self-emulsifying emulsion can be prepared by a method including uniformly mixing a phospholipid and/or a lysophospholipid and a polyhydric alcohol under stirring, and then further mixing and stirring the mixture while pouring a fat or oil.

The oil component used in the self-emulsifying emulsion may be any oil component that has no adverse effect on a human body. The oil component may be, for example, a vegetable oil, such as soybean oil, sesame oil, rapeseed oil, safflower oil, olive oil, castor oil, corn oil, cottonseed oil, rice oil, sunflower oil, grape seed oil, or wheat germ oil; medium-chain triglyceride (MCT); etc. The amount of the oil component contained in the self-emulsifying emulsion is not particularly limited, and may be, for example, from 0.1% by mass to 35% by mass based on the total mass of the formulation. When the oil component is in this range, the self-emulsifying emulsion can easily form a nano-scale oil-in-water emulsion upon contact with a body fluid, such as blood or a digestive juice, and the effect of the present invention can be easily exhibited.

The polyhydric alcohol used in the self-emulsifying emulsion may be any polyhydric alcohol that has no adverse effect on a human body. The polyhydric alcohol may be, for example, 1,3-butanediol, 1,3-propanediol, 3-methyl-1,3-butanediol, propylene glycol, dipropylene glycol, polypropylene glycol, butylene glycol, 1,2-pentanediol, ethylene glycol, diethylene glycol, triethylene glycol, polyethylene glycol, polyoxyethylene glycol, glycerol, diglycerol, polyglycerol such as tetraglycerol, xylitol, maltitol, pentaerythritol, sorbitol, trehalose, etc. Among them, glycerol, propylene glycol and butylene glycol are preferred due to their high safety to a human body. The amount of the polyhydric alcohol is not particularly limited, but when the amount of the polyhydric alcohol is, for example, from 20% by mass to 99% by mass based on the total mass of the formulation, the self-emulsifying emulsion can easily form a nano-scale oil-in-water emulsion upon contact with a body fluid, such as blood or a digestive juice, and the effect of the present invention can be easily exhibited.

Phospholipids and lysophospholipids are substances found in living bodies and have been used as active pharmaceutical ingredients or additives for administration to humans. Phospholipids and lysophospholipids have low toxicity to humans and other mammals (e.g., rats, mice, rabbits, sheep, pigs, cows, cats, dogs, monkeys, etc.) and can be safely administered. The amount of the active ingredient in the formulation is determined as appropriate for the dosage form, the mode of administration, the carrier used, etc., and typically 0.01 to 100% by mass, preferably 0.1 to 95% by mass of a phospholipid and/or a lysophospholipid can be added based on the total amount of the formulation.

The dosage of the phospholipid and/or the lysophospholipid may vary with the subject of administration, the symptoms, the route of administration, etc. For oral administration, the dosage for a human with a body weight of about 60 kg is generally, for example, from about 0.01 to 25000 mg per day, preferably from about 0.1 to 2500 mg per day, and more preferably from about 0.5 to 500 mg per day. For parenteral administration, the daily dosage may vary with the condition and symptoms of a patient, the mode of administration, etc. For example, for an injection, the phospholipid and/or the lysophospholipid is typically administered, for example, at a dose of from about 0.01 to 100 mg per kg body weight, preferably from about 0.01 to 50 mg per kg body weight, and more preferably from about 0.01 to 20 mg per kg body weight.

The present invention further includes the following.

A method for preventing or ameliorating cachexia, comprising administering an effective amount of a phospholipid and/or a lysophospholipid to a mammal.

A phospholipid and/or a lysophospholipid for use in preventing or ameliorating cachexia.

Use of a phospholipid and/or a lysophospholipid for preventing or ameliorating cachexia.

Use of a phospholipid and/or a lysophospholipid for production of a preventing or ameliorating agent for cachexia.

### Method for screening for substance for ameliorating cachexia

The present invention provides a screening method for a substance for ameliorating cachexia. A first embodiment of the screening method of the present invention is a method comprising selecting a test substance that promotes myotube formation as compared to that without addition of the test substance when culture is performed in an in vitro differentiation induction system of a stem cell or a progenitor cell into a skeletal muscle cell using a differentiation induction medium containing a culture supernatant of a cancer cell with addition of the test substance. A second embodiment of the screening method of the present invention is a method comprising selecting a test substance that promotes adipocyte differentiation as compared to that without addition of the test substance when culture is performed in an in vitro differentiation induction system of a stem cell or a progenitor cell into an adipocyte using a differentiation induction medium containing a culture supernatant of a cancer cell with addition of the test substance.

The test substance subjected to the screening method of the present invention is not particularly limited and may be, for example, a naturally occurring compound, an organic compound, an inorganic compound, a nucleic acid, a nucleic acid oligo, a protein, a peptide, a cell extract, a cell culture supernatant, a product from a fermentation microorganism, an extract from a marine organism, a plant extract, a prokaryotic cell extract, an extract from a unicellular eukaryotic organism, an animal cell extract, etc. The test substance may be an expression product or the like from a compound library, a nucleic acid oligo library, a peptide library, or a gene library. The test substance may be a novel substance or a known substance. The test substance may be in the form of a salt. The salt of the test substance is preferably a salt with a physiologically acceptable acid or base.

The culture supernatant of a cancer cell used in the screening method of the present invention may be any culture supernatant of a cancer cell cultured by a conventional method, and is not particularly limited. The culture supernatant is preferably a culture supernatant of a cancer cell that has been seeded under normal conditions and cultured to near confluency (about 80% or more confluency). The type of cancer cell used is not particularly limited, but preferably a cancer cell of the same cell type as the stem or progenitor cell used in the screening method.

The stem cell or progenitor cell used in the first embodiment is any type of cell capable of differentiating into a skeletal muscle cell, and may be, for example, a skeletal muscle stem cell (satellite cell), a myoblast, etc. The differentiation induction medium used to induce differentiation into a skeletal muscle cell may be selected from known media that can be used for the induction of differentiation of stem cells or progenitor cells into skeletal muscle cells. For example, a medium containing horse serum, basic fibroblast growth factor (bFGF), hepatocyte growth factor (HGF), and/or the like can be used.

In the first embodiment, a stem cell or a progenitor cell is induced to differentiate into a skeletal muscle cell in a differentiation induction medium containing the culture supernatant of a cancer cell with addition of a test substance (test substance group), and myotube formation in the presence of the test substance is compared to that in a control group without addition of the test substance to select a test substance that promotes myotube formation as a substance that ameliorates cachexia. The myotube formation can be evaluated by, for example, observing the cultured cells under a microscope.

The stem cell or progenitor cell used in the second embodiment is any type of cell capable of differentiating into an adipocyte, and may be, for example, a mesenchymal stem cell, an adipocyte progenitor cell, etc. The differentiation induction medium used to induce differentiation into an adipocyte may be selected from known media that can be used for the induction of differentiation of stem cells or progenitor cells into adipocytes. For example, a medium containing insulin, dexamethasone, and/or the like can be used.

In the second embodiment, a stem cell or a progenitor cell is induced to differentiate into an adipocyte in a differentiation induction medium containing the culture supernatant of a cancer cell with addition of a test substance (test substance group), and adipocyte differentiation in the presence of the test substance is compared to that in a control group without addition of the test substance to select a test substance that promotes adipocyte differentiation as a substance that ameliorates cachexia. The promotion of differentiation into an adipocyte can be evaluated by, for example, staining the cultured cells with Oil Red O, and counting the number of positive cells.

### EXAMPLES

The present invention will be described in more detail below with reference to Examples, but the present invention is not limited thereto.

### Example 1: Effect of PC and LPC on in vitro model of skeletal muscle mass loss induced by cachexic substance

### 1-1 Experimental method

### (1) Cells used

Mouse myoblasts (C2C12, RIKEN cell bank) and mouse colon cancer cells (Colon26, RIKEN cell bank) were used. The culture medium used was DMEM medium (Sigma-Aldrich Japan) or RPMI medium (Sigma-Aldrich Japan) supplemented with 10% fetal bovine serum (Gibco), 100 units/mL penicillin G (Sigma-Aldrich Japan) and 100 µg/mL streptomycin (Sigma-Aldrich Japan).

### (2) Preparation of culture supernatant of Colon26 cells

Colon26 cells were seeded at a concentration of 5 × 10⁴ cells/cm² in a culture dish with a diameter of 10 mm, and cultured at 37°C, 5% CO₂ to 80% to 90% confluency. The medium was then changed to a medium supplemented with 16:0 lysophosphatidylcholine (hereinafter referred to as LPC), 18:0 LPC, 16:0 phosphatidylcholine (hereinafter referred to as PC), or 18:0 PC (all manufactured by Avanti Polar Lipids) at a final concentration of 25 µM, or a medium not containing LPC or PC. The cells were cultured for another 24 hours. After the cells were washed with PBS, the medium was changed to serum-free RPMI medium, and the cells were cultured for another 48 hours. The medium was collected and centrifuged at 4°C and 3000 rpm for 20 minutes to precipitate the cellular components. The supernatant was sterile-filtered using a 0.22 µm sterile filter (Merck Millipore) and stored at -80°C until use.

### (3) Culture of C2C12 cells

C2C12 cells were cultured to 80% to 90% confluency in a 6-well culture plate. The medium was changed to DMEM medium supplemented with horse serum (Gibco, final concentration: 2%) or horse serum-supplemented DMEM medium containing 50% supernatant of one of the five types prepared in the above (2), and the cells were cultured for 9 days to induce differentiation into skeletal muscle cells. On day 9, the morphology of the cells was observed under a phase-contrast microscope.

### 1-2 Results

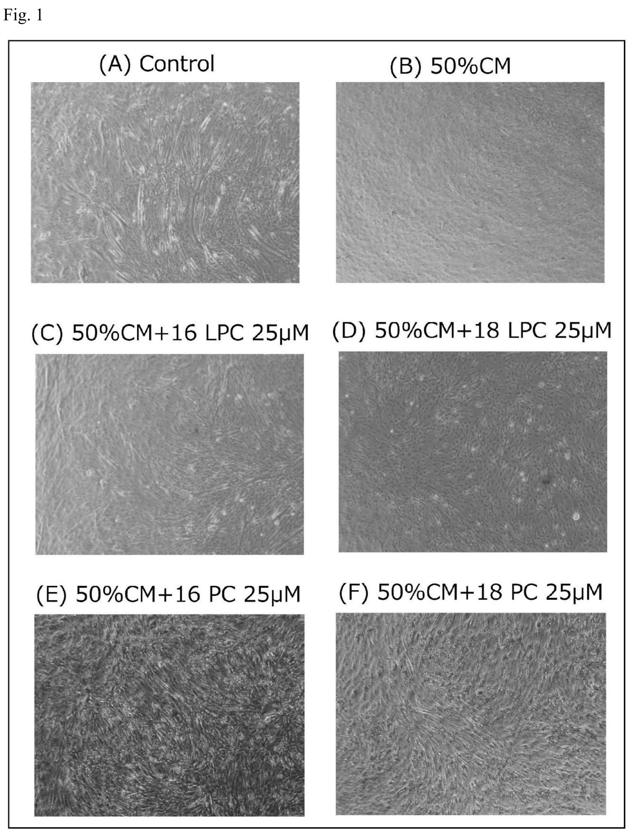

Fig. 1 shows the results. (A) Control shows an image of the cells cultured in the DMEM medium supplemented with horse serum. (B) 50% CM shows an image of the cells cultured in the horse serum-supplemented DMEM medium containing 50% supernatant of Colon26 cells. (C) 50% CM + 16 LPC 25 µM shows an image of the cells cultured in the horse serum-supplemented DMEM medium containing 50% supernatant of Colon26 cells supplemented with 16:0 LPC. (D) 50% CM + 18 LPC 25 µM shows an image of the cells cultured in the horse serum-supplemented DMEM medium containing 50% supernatant of Colon26 cells supplemented with 18:0 LPC. (E) 50% CM + 16 LPC 25 µM shows an image of the cells cultured in the horse serum-supplemented DMEM medium containing 50% supernatant of Colon26 cells supplemented with 16:0 PC. (F) 50% CM + 18 PC 25 µM shows an image of the cells cultured in the horse serum-supplemented DMEM medium containing 50% supernatant of Colon26 cells supplemented with 18:0 PC. In (A) Control, myotube formation of skeletal muscle cells differentiated from the myoblasts was observed, but myotube formation was inhibited in the cells (B) cultured in the medium containing the culture supernatant of Colon26 cells. The inhibition of differentiation of the myoblasts into skeletal muscle cells was prevented in the cells (C) and (D) cultured in the medium containing the culture supernatant of Colon26 cells supplemented with LPC and in the cells (E) and (F) cultured in the medium containing the culture supernatant of Colon26 cells supplemented with PC, although there was a difference in the degree of prevention, and myotube formation was observed.

These results indicate that a cachexic substance that inhibits the differentiation of myoblasts into skeletal muscle cells is contained in the factors secreted from cancer cells. In other words, the results reveal that the cachexic substance secreted from cancer cells induces skeletal muscle mass loss, and this culture system seemed to serve as an in vitro model of skeletal muscle mass loss due to a cachexic substance. The results also confirm that when cancer cells receive a stimulus from LPC or PC using this culture system, the expression of the skeletal muscle mass loss-inducing factor (a cachexic substance) is inhibited.

### Example 2: Effect of LPC on in vitro model of adipocyte atrophy induced by cachexia

### 2-1 Experimental method

### (1) Cells used

Mesenchymal OP9 cells (RIKEN cell bank) were used. The culture medium used was MEM medium (Sigma-Aldrich Japan) supplemented with 20% fetal bovine serum (Gibco), 100 units/mL penicillin G (Sigma-Aldrich Japan), 100 µg/mL streptomycin (Sigma-Aldrich Japan), and 2 mM L-glutamine.

### (2) Culture of OP9 cells

OP9 cells were seeded at a concentration of 7 × 10³ cells/cm² in a 96-well culture plate and cultured to confluency. After the cells reached confluency, the medium was changed to an adipocyte differentiation medium prepared by adding KnockOut SR (Invitrogen) at a concentration of 15% to MEM medium supplemented with 100 units/mL penicillin G and 100 µg/mL streptomycin; the adipocyte differentiation medium further containing 10% supernatant of Colon26 cells prepared in Example 1 (2); or the adipocyte differentiation medium further containing 10% supernatant of Colon26 cells supplemented with 16:0 LPC, to promote the differentiation of OP9 cells into adipocytes. The medium was replaced every 4 days, and the cells were cultured for 7 days. On day 7, OP9 cells were fixed in 4% paraformaldehyde (Wako) for 30 to 60 minutes, washed twice with 60% isopropanol, stained with Oil Red O Solution (Cayman Chemical) for 20 minutes, and observed under a microscope.

### 2-2 Results

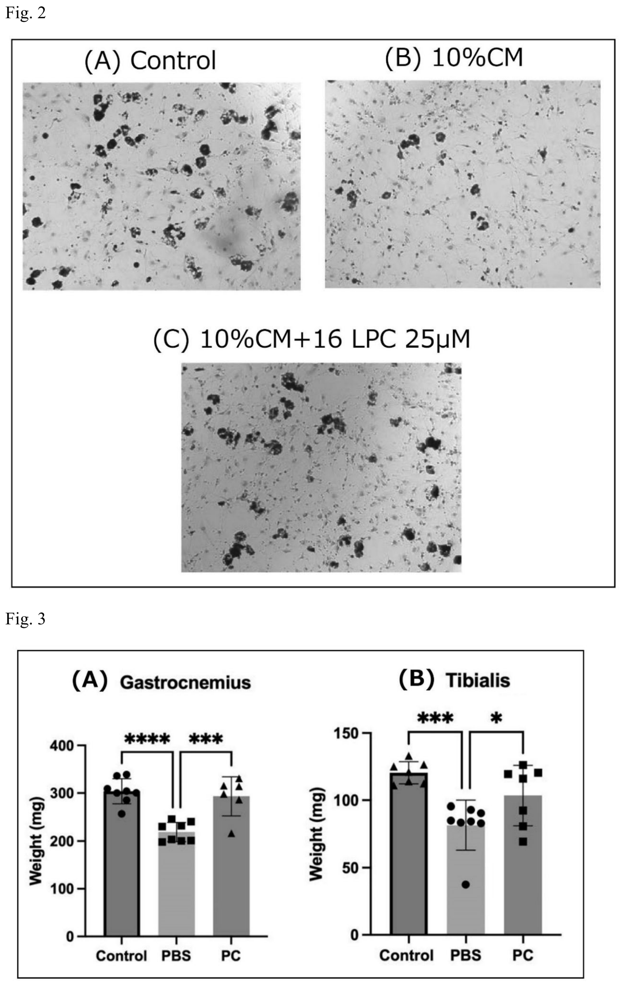

Fig. 2 shows the results. (A) Control shows an image of the cells cultured in the adipocyte differentiation medium. (B) 10% CM shows an image of the cells cultured in the adipocyte differentiation medium containing 10% supernatant of Colon26 cells. (C) 10% CM + 16 LPC 25 µM shows an image of the cells cultured in the adipocyte differentiation medium containing 10% supernatant of Colon26 cells supplemented with 16:0 LPC. In (A) Control, adipocytes stained with Oil Red O were observed, but adipocyte differentiation was inhibited in the cells (B) cultured in the medium containing the culture supernatant of Colon26 cells. The inhibition of adipocyte differentiation was prevented in the cells (C) cultured in the medium containing the culture supernatant of Colon26 cells supplemented with LPC, and adipocytes stained with Oil Red O were observed. These results indicate that a cachexic substance that inhibits the differentiation and maintenance of adipocytes is contained in the molecules secreted from cancer cells, and that LPC inhibits the secretion of such a cachexic substance.

### Example 3: Effect of PC administration on skeletal muscle mass loss in cancer-bearing mice

### 3-1 Experimental method

### (1) Animals used and transplantation of cancer cells

Male BalB/c mice (SLC) at 8 to 9 weeks old were used. Colon26 cells were cultured to 80% to 90% confluency in the same manner as in Example 1 and suspended in PBS to a concentration of 1 × 10⁶ cells/mL. Then, 100 µL of the cell suspension was subcutaneously transplanted into the right upper flank of the mice. Every 3 or 4 days, the body weight of the mice was measured, and the general conditions of the mice were observed. The tumor size [1/2(a × b²) mm³] was calculated by measuring the maximum diameter (a) and the minimum diameter (b) of the tumor.

### (2) Preparation of phosphatidylcholine (PC)

Hydrogenated PC (HSPC; Avanti Polar Lipids) was dissolved in ethanol to a concentration of 50 mM and stored at -20°C until use.

### (3) Grouping and dosing schedule

We set up three groups of mice: a non-cancer cell transplantation group (control), a cancer cell transplantation group with administration of a solvent (PBS), and a cancer cell transplantation group with administration of PC (PC). One week after the transplantation of Colon26 cells, the mice were randomly divided into groups of at least five mice, and the administration of PC or a solvent (PBS) was started. PC and the solvent were administered via the tail vein every 3 or 4 days. The dose of PC was set to 3 mg/kg (body weight). PC was diluted in PBS and administered at a volume of 100 µL per mouse. The solvent (PBS) was administered at a volume of 100 µL per mouse.

### (4) Measurement of muscle weight and quantitative PCR

The mice were euthanized on day 36 post-transplantation of Colon26 cells, and the gastrocnemius and tibialis muscles were harvested to measure the weight. Twenty milligrams of each muscle tissue was homogenized using High Power Homogenizer ASG50 (AS ONE Corporation), and the total RNA was recovered using RNeasy Fibrous Mini Kit (Qiagen). cDNA was synthesized from the total RNA using PrimeScript RT reagent Kit (Takara) and subjected to quantitative PCR (qPCR). The primer sets shown below were used to detect the MuRF-1 gene and the Atrogin-1 gene, which are known to induce the degradation of muscle. The qPCR reagent was TB Green Premix Ex Taq II (Takara), and the real-time PCR device was Light Cycler 96 system (Roche).

Primer set for amplification of mouse GAPDH gene:
sense 5'-TGATACAGTCGGGGTGAGTG-3' (SEQ ID NO: 1), and
anti-sense 5'-AAGATGGTGATGGGCTTCCCG-3' (SEQ ID NO: 2).

Primer set for amplification of mouse MuRF-1 gene:
sense 5'-ACGGAAACGACCTCCAGACATG-3' (SEQ ID NO: 3), and
anti-sense 5'-TACCAAGCCTGTGGTCATCCTG-3' (SEQ ID NO: 4).

Primer set for amplification of mouse Atrogin-1 gene:
sense 5'-CTTCTCGACTGCCATCCTGGAT-3' (SEQ ID NO: 5), and
anti-sense 5'-TCTTTTGGGCGATGCCACTCAG-3' (SEQ ID NO: 6).

### 3-2 Results

Fig. 3 shows the results of measuring the muscle weight. The panel (A) shows the weight of the gastrocnemius muscle (Gastrocnemius), and the panel (B) shows the weight of the tibialis muscle (Tibialis). In the solvent administration group (PBS), the muscle weight decreased in both the gastrocnemius muscle and the tibialis muscle, indicating that the skeletal muscle mass loss was induced by cachexia. However, in the PC administration group (PC), muscle weight loss was not observed in the gastrocnemius or tibialis muscle, revealing that there was no induction of skeletal muscle mass loss by cachexia.

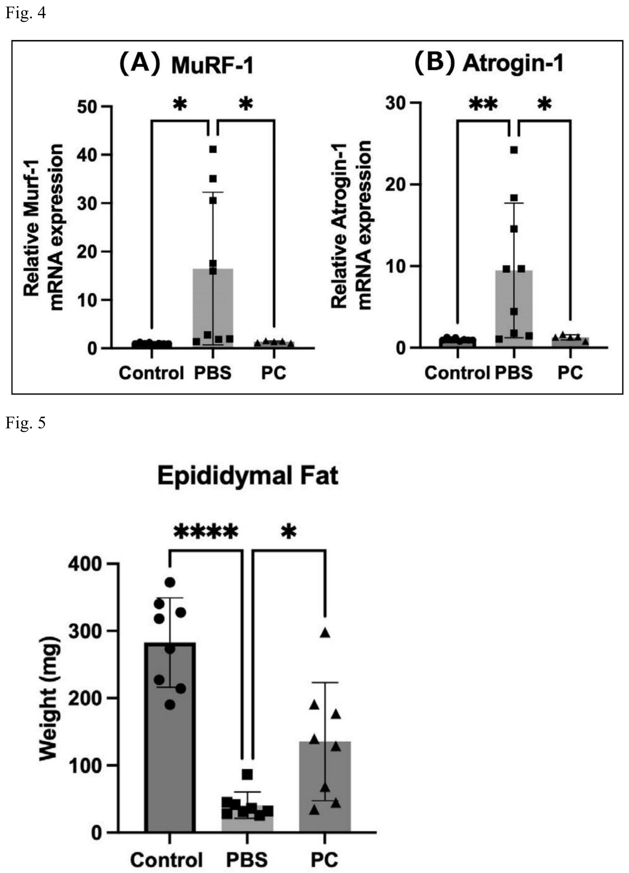

Fig. 4 shows the results of quantitative PCR. The panel (A) shows the result of qPCR of the MuRF-1 gene, and the panel (B) shows the result of qPCR of the Atrogin-1 gene. The expression of both the MuRF-1 gene and the Atrogin-1 gene was enhanced in the solvent administration group (PBS), whereas the expression of both genes in the PC administration group (PC) was comparable to that in normal mice. These results were consistent with the results of measuring the muscle weight.

### Example 4: Effect of PC administration on survival rate and lipoatrophy in cancer-bearing mice 4-1 Experimental method

Mice were transplanted with Colon26 cells in the same manner as in Example 3, and one week later, divided into three groups: a non-cancer cell transplantation group (control), a cancer cell transplantation group with administration of a solvent (PBS, n = 20), and a cancer cell transplantation group with administration of PC (PC, n = 20). PC and a solvent (PBS) were administered by the same dosing schedule as that in Example 3. During the dosing period, the general conditions of the mice were observed every day. The survival rate was calculated by counting the number of dead individuals. The surviving mice were euthanized on day 36 post-transplantation of Colon26 cells, and the epididymal fat tissue was harvested to measure the weight (groups of n = 8 each). Total RNA was recovered from less than 100 mg of the epididymal fat tissue, and cDNA was synthesized and subjected to qPCR in the same manner as in Example 3 (groups of n = 2 each). The reagents and devices were the same as those used in Example 3. The primer set shown below was used to detect the Leptin gene, which is known to have the function of maintaining fat tissue. The primer set for amplification of the mouse GAPDH gene was the same as that used in Example 3.

Primer set for amplification of mouse Leptin gene:
sense 5'-TGAGTTTGTCCAAGATGGACC-3' (SEQ ID NO: 7), and
anti-sense 5'-GCCATCCAGGCTCTCTGG-3' (SEQ ID NO: 8).

### 4-2 Results

Fig. 5 shows the result of measuring the weight of the epididymal fat tissue on day 36 post-transplantation of the cancer cells. In the solvent administration group (PBS), the weight of the fat tissue decreased, indicating that lipoatrophy was induced by cachexia. However, in the PC administration group (PC), weight loss in the fat tissue was not observed, revealing that lipoatrophy was inhibited.

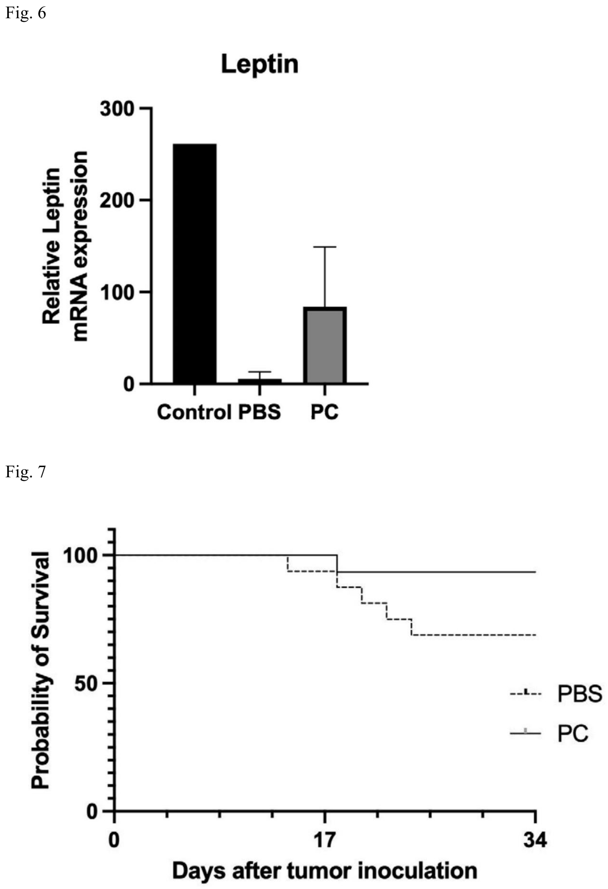

Fig. 6 shows the result of quantitative PCR. The expression of the Leptin gene was inhibited in the solvent administration group (PBS), in which lipoatrophy was induced. However, the expression of the Leptin gene in the PC administration group (PC), in which lipoatrophy was inhibited, was comparable to that in normal mice.

Fig. 7 shows the survival rate. The survival rate in the PC administration group (PC) was improved as compared to that in the solvent administration group (PBS).

### Example 5: Effect of administration of PC, LPC or PC/LPC mixture on cancer-bearing mice 1

### 5-1 Experimental method

### (1) Animals used, transplantation of cancer cells and grouping

Mice were transplanted with Colon26 cells in the same manner as in Example 3, and one week later, divided into six groups: a non-cancer cell transplantation group (control), a cancer cell transplantation group with administration of a solvent (PBS), a cancer cell transplantation A group (PC A), a cancer cell transplantation B group (PC B), a cancer cell transplantation C group (PC C), and a cancer cell transplantation D group (PC D). The mice were randomly divided into groups of at least five mice.

### (2) Preparation of PC, LPC and PC/LPC mixture (self-emulsifying emulsion) and dosing schedule

PC was a phosphatidylcholine extracted from egg yolk (Egg Yolk Lecithin PC-98N, Kewpie Corporation). LPC was a lysophosphatidylcholine extracted and purified from egg yolk treated with phospholipase. The A group received a mixture of PC:LPC = 1:2 (self-emulsifying emulsion). The B group received a mixture of PC:LPC = 1:4 (self-emulsifying emulsion). The C group received PC only. The D group received LPC only. The mixture of PC and LPC was prepared by uniformly mixing PC and LPC and propylene glycol under stirring, and pouring and mixing a medium-chain fatty acid oil (COCONARD RK, Kao Chemical) into the mixture under stirring. The mass ratio of the components in the mixture for the A group is PC:LPC:medium-chain fatty acid oil:propylene glycol = 16.7:33.3:5.0:45.0. The mass ratio of the components in the mixture for the B group is PC:LPC:medium-chain fatty acid oil:propylene glycol = 10.0:40.0:5.0:45.0. The sum of the doses of PC and LPC was set to 3 mg/kg. A volume of 100 µL was orally administered using a mouse gavage needle (FCR & Bio) every three or four days starting from one week after transplantation of the cancer cells.

### (3) Measurement of muscle weight, muscle tissue analysis, quantitative PCR and measurement of serum IL-6 levels

On day 24 post-transplantation of Colon26 cells, the mice were anesthetized, and the blood was collected from the heart using a Microtainer serum separator tube (BD). The mice were then euthanized, and the gastrocnemius muscle and the tibialis muscle were harvested to measure the weight and stored in liquid nitrogen. The gastrocnemius muscle of the three mice randomly selected from each group was thawed, fixed in 4% paraformaldehyde for 48 hours, and embedded in paraffin. Histological sections of 4 µm thickness were prepared and stained with hematoxylin and eosin (Wako). The specimens were photographed at a magnification of ×20 using a VS-200 research slide scanner (Olympus). Image analysis was performed using QuPath software and Fiji software (Image J), and the circumference of muscle fibers was measured for 50 fibers per mouse (n = 3). The expression levels of the MuRF-1 and Atrogin-1 genes were also determined by quantitative PCR in the same manner as in Example 3. The collected blood was allowed to stand at room temperature for 30 minutes and centrifuged at 9000 rpm for 10 minutes to recover the serum. The serum IL-6 levels were measured using LEGEND MAX Mouse IL-6 ELISA Kit (BioLegend).

### 5-2 Results

### (1) Muscle weight

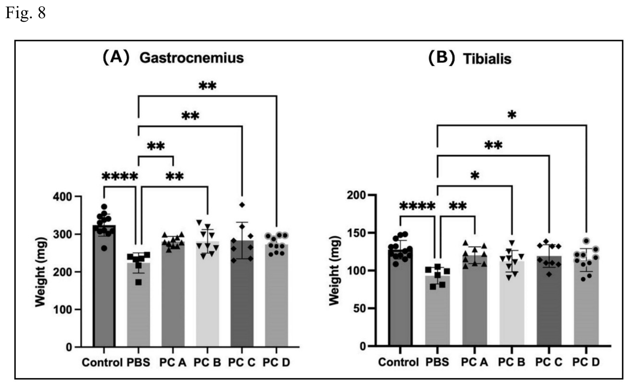

Fig. 8 shows the results of measuring the muscle weight. The panel (A) shows the weight of the gastrocnemius muscle (Gastrocnemius), and the panel (B) shows the weight of the tibialis muscle (Tibialis). In the solvent administration group (PBS), the muscle weight decreased in both the gastrocnemius muscle and the tibialis muscle, indicating that the skeletal muscle mass loss was induced by cachexia. However, in the A, B, C and D groups, muscle weight loss was not observed in the gastrocnemius or tibialis muscle, revealing that skeletal muscle mass loss induced by cachexia was inhibited.

### (2) Histological images of muscle and the circumference of muscle fibers

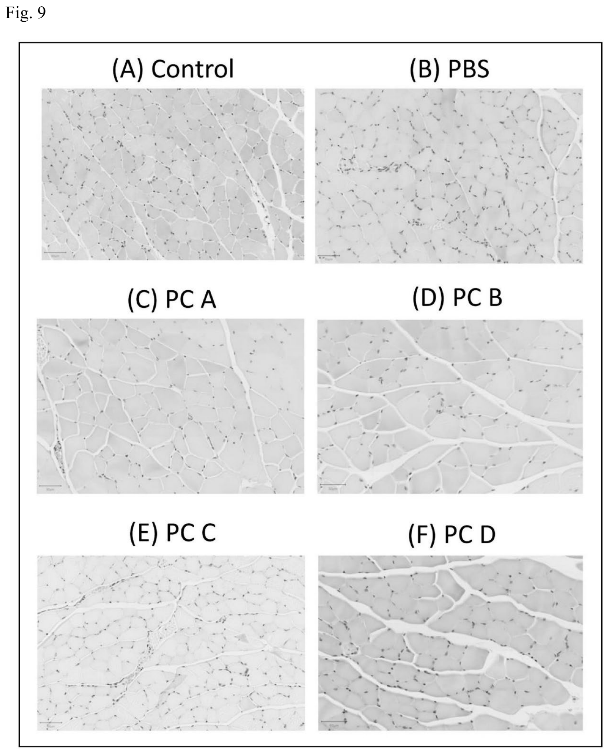

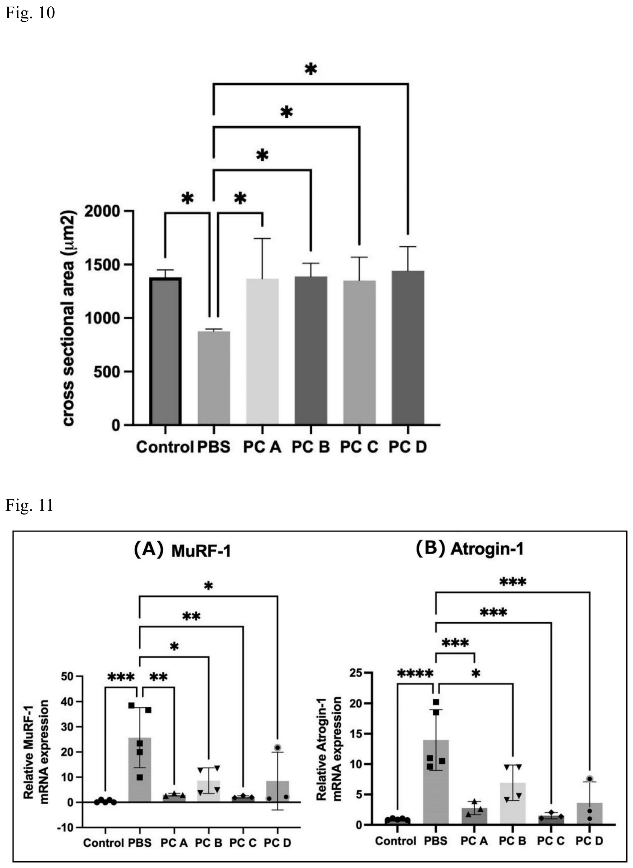

Fig. 9 shows the histological images of the gastrocnemius muscle. Fig. 10 shows the analysis result by comparison of the circumference of muscle fibers measured on the histological images of the muscle among the groups. Atrophy of the muscle fibers induced by cachexia was inhibited in the A, B, C and D groups.

### (3) Quantitative PCR

Fig. 11 shows the results of quantitative PCR. The panel (A) shows the result of qPCR of the MuRF-1 gene, and the panel (B) shows the result of qPCR of the Atrogin-1 gene. The expression of both the MuRF-1 and Atrogin-1 genes, which induce the degradation of muscle, was enhanced in the solvent administration group (PBS). However, the expression of both genes was inhibited in the A, B, C and D groups.

### (4) Serum IL-6 levels

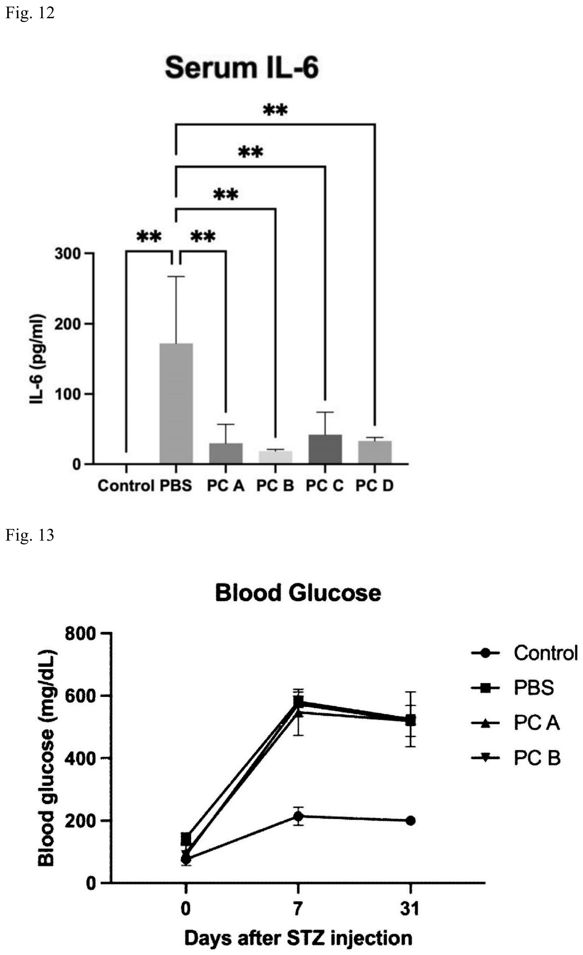

Fig. 12 shows the serum IL-6 levels. The serum IL-6 levels were significantly increased in the solvent administration group (PBS). However, the increase in the serum IL-6 levels was inhibited in the A, B, C and D groups. These results reveal that the inhibitory effect of PC, LPC or the PC/LPC mixture on cachexia is not limited to muscles or fat tissue but is also exhibited on systemic inflammation.

### Example 6: Effect of administration of PC/LPC mixture on type 1 diabetic model mice

### 6-1 Experimental method

### (1) Animals used and type 1 diabetic model mice

Male C57BL/6J mice (SLC) at 8 weeks old were used. The mice were preliminarily maintained for one week or longer to acclimate to the environment and fasted for 14 hours to starvation. Streptozocin (STZ) was dissolved in 0.1 M citrate buffer (pH 4.5) stored at a low temperature, and intraperitoneally administered at 150 mg/kg over 15 minutes. To control mice, the citrate buffer alone was intraperitoneally administered at the same volume. Every 3 or 4 days, the body weight of the mice was measured, and the general conditions of the mice were observed. On day 7 after the administration of STZ, the blood glucose levels were measured using a lab glucometer (Fora). The animals with a blood glucose level of 250 mg/dL or more were selected and used as a diabetic model for the subsequent experiments.

### (2) Grouping and dosing schedule

The mice were divided into four groups: a non-diabetic group (control), a diabetic group with administration of a solvent (PBS), a diabetic A group (PC A), and a diabetic B group (PC B). The A group received a mixture of PC:LPC = 1:2, and the B group received a mixture of PC:LPC = 1:4 (see Example 5). The dose of the PC/LPC mixture was set to 3 mg/kg. A volume of 100 µL was orally administered using a mouse gavage needle (FCR & Bio) every three or four days during days 7 to 31 post-administration of STZ.

### (3) Measurement of blood glucose levels, measurement of muscle weight and quantitative PCR

On day 31 after the administration of STZ, the blood glucose levels were measured using a lab glucometer (Fora) in the same manner as on day 7. The mice were then euthanized, and the gastrocnemius and tibialis muscles were harvested to measure the weight. The expression levels of the MuRF-1 and Atrogin-1 genes were determined by quantitative PCR in the same manner as in Example 3.

### 6-2 Results

### (1) Blood glucose levels

Fig. 13 shows the result of measuring the blood glucose levels. The blood glucose levels of the mice in the non-diabetic group (control) persisted at the level of about 200 mg/dL, whereas the blood glucose levels of the mice in the diabetic group with administration of a solvent (PBS) persisted at the level of about 600 mg/dL. The blood glucose levels of the diabetic A group (PC A) and the diabetic B group (PC B), which received oral administration of the PC/LPC mixture, were comparable to that of the diabetic group with administration of a solvent (PBS), and no significant difference was observed among the groups.

### (2) Muscle weight

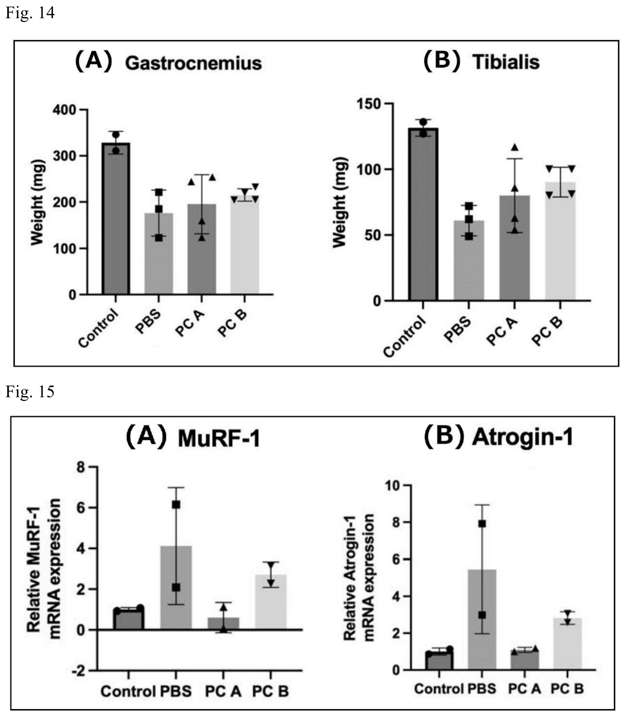

Fig. 14 shows the results of measuring the muscle weight. The panel (A) shows the weight of the gastrocnemius muscle (Gastrocnemius), and the panel (B) shows the weight of the tibialis muscle (Tibialis). In the solvent administration group (PBS), the muscle weight decreased in both the gastrocnemius and tibialis muscles. However, the degree of loss of the muscle weight was lower in the diabetic A group (PC A) and the diabetic B group (PC B), which received oral administration of the PC/LPC mixture.

### (3) Quantitative PCR

Fig. 15 shows the results of quantitative PCR. The panel (A) shows the result of qPCR of the MuRF-1 gene, and the panel (B) shows the result of qPCR of the Atrogin-1 gene. The expression of both the MuRF-1 and Atrogin-1 genes, which induce the degradation of muscle, was enhanced in the solvent administration group (PBS). However, the expression of both genes was inhibited in the diabetic A group (PC A) and the diabetic B group (PC B), which received oral administration of the PC/LPC mixture.

### Example 7: Effect of administration of PC, LPC or PC/LPC mixture on cancer-bearing mice (2)

### 7-1 Experimental method

### (1) Measurement of fat tissue weight and fat tissue analysis

The experiment was performed in the same manner as in Example 5. On day 24 post-transplantation of Colon26 cells, the mice were anesthetized, and the blood was collected from the heart using a Microtainer serum separator tube (BD). The mice were then euthanized, and the appearance of the epididymal fat tissue was photographed. The epididymal fat tissue was then harvested to measure the weight and stored in liquid nitrogen. The epididymal fat tissue of the three mice randomly selected from each group was thawed, fixed in 4% paraformaldehyde for 48 hours, and embedded in paraffin. Histological sections of 4 µm thickness were prepared and stained with hematoxylin and eosin (Wako). The specimens were photographed at a magnification of ×20 using a VS-200 research slide scanner (Olympus). Image analysis was performed using QuPath software and Fiji software (Image J), and the circumference of adipocytes was measured for 50 cells in the fat tissue per mouse (n = 3). The collected blood was allowed to stand at room temperature for 30 minutes and centrifuged at 9000 rpm for 10 minutes to recover the serum. The serum oncostatin M (OSM: Oncostatin M) levels were measured using Mouse Oncostatin M (OSM) Quantikine ELISA Kit (R&D Systems).

### 7-2 Results

### (1) Appearance and weight of fat tissue

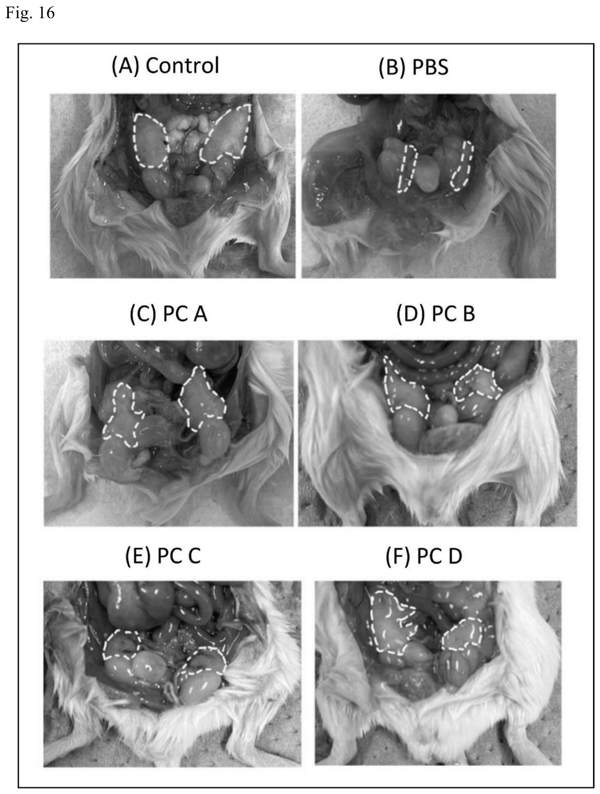

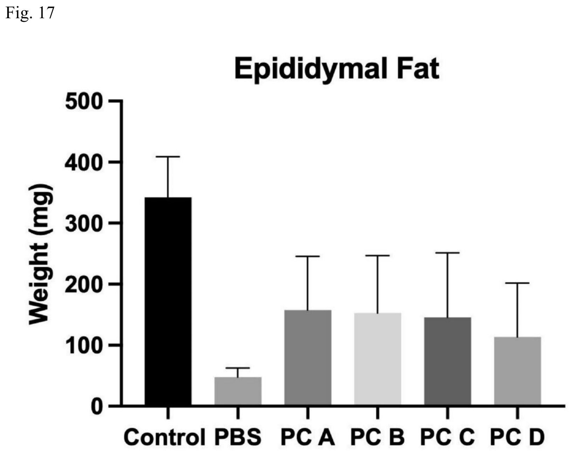

Fig. 16 shows the appearance of the epididymal fat tissue. Fig. 17 shows the result of measuring the fat tissue weight. In the solvent administration group (PBS), the weight of the fat tissue decreased, indicating that lipoatrophy was induced by cachexia. However, the loss of fat tissue weight induced by cachexia was inhibited in the A, B, C and D groups.

### (2) Histological images of fat tissue and the circumference of adipocytes

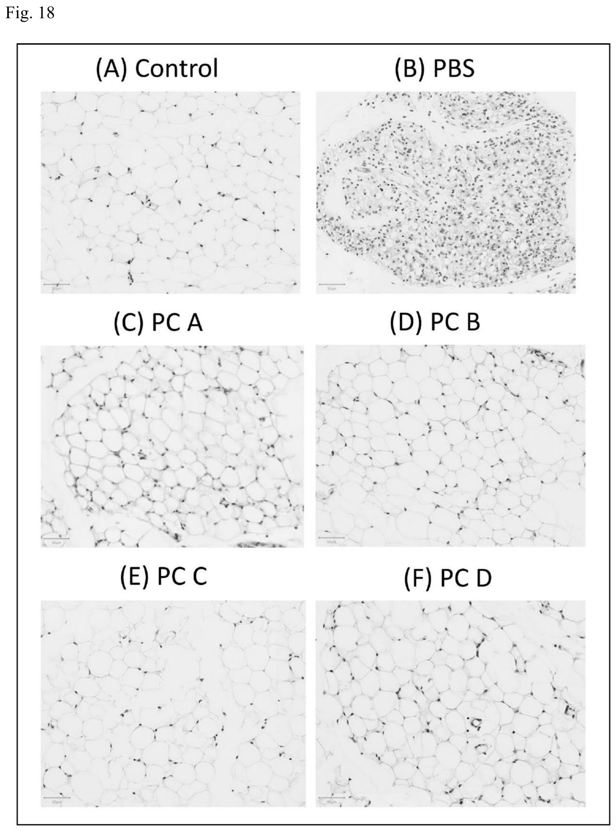

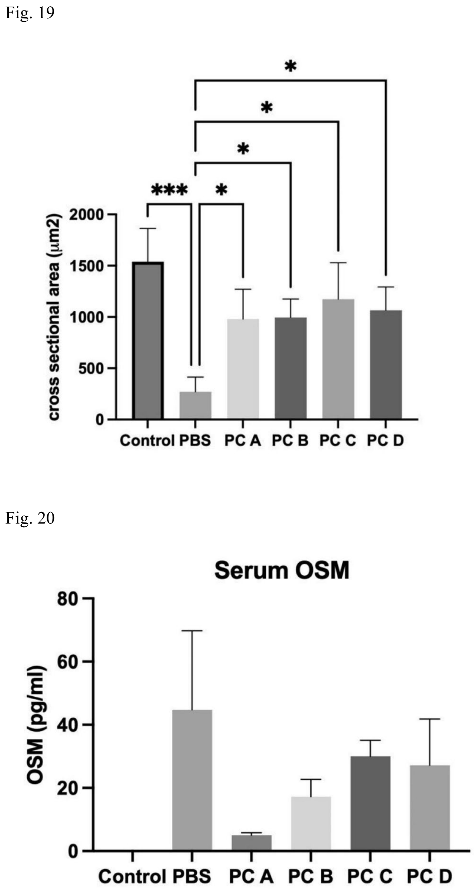

Fig. 18 shows the histological images of the epididymal fat tissue. Fig. 19 shows the result of measuring the circumference of individual adipocytes. The loss of lipid droplets in adipocytes was inhibited, and a decrease in the circumference of adipocytes was also inhibited in the A, B, C and D groups. These results revealed that lipoatrophy induced by cachexia is due to the inhibition of the loss of lipid droplets in adipocytes.

### (3) Serum oncostatin M (OSM) levels

Fig. 20 shows the serum OSM levels. The serum OSM levels were significantly increased in the solvent administration group (PBS). However, the increase in the serum OSM levels was inhibited in the A, B, C and D groups. OSM is believed to serve as a cytokine that promotes cancer cachexia. The results revealed that PC, LPC and a PC/LPC mixture inhibit the secretion of OSM, thereby inhibiting cancer cachexia.

### Conclusion

Taken together, the results revealed that PC and LPC ameliorate not only cachexia induced by cancer but also cachexia induced by other metabolic and inflammatory diseases, such as diabetes mellitus.

### Example 8: Effect of PC and PS on enhanced expression of IL-6 induced by cachexic substance 8-1 Experimental method

Colon26 cells were seeded at a concentration of 5 × 10⁴ cells/cm² in the medium used in Example 1 in a culture dish with a diameter of 10 mm, and cultured at 37°C, 5% CO₂ to 80% to 90% confluency. To the culture dish, 16:0 PC or 18:0 PC was added at a final concentration of 25 µM, and PS (Larodan) was added at a final concentration of 100 µg/ml. The cells were cultured for 24 hours. After the cells were washed with PBS, the medium was changed to serum-free RPMI medium (Wako), and the cells were cultured for another 48 hours. The medium was collected and centrifuged at 4°C and 3000 rpm for 20 minutes to precipitate the cellular components. The supernatant was filtered through a 0.22 µm sterile filter to remove precipitates from the supernatant. The serum IL-6 level in the supernatant was measured using LEGEND MAX Mouse IL-6 ELISA Kit (BioLegend).

### 8-2 Results

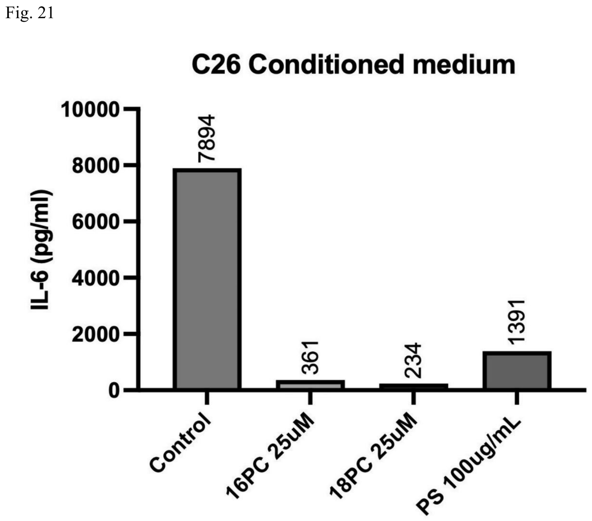

Fig. 21 shows the result. The result reveals that 16:0 PC, 18:0 PC, and PS have a direct inhibitory effect on the expression of IL-6 in cancer cells. This finding demonstrates that PC has an inhibitory effect on cachexia regardless of the number of carbon atoms that form PC, and that not only PC but also phospholipids like PS inhibit cachexia.

The present invention is not limited to each of the embodiments or Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A preventing or ameliorating agent for cachexia, comprising a phospholipid and/or a lysophospholipid as an active ingredient.

2. The preventing or ameliorating agent according to claim 1, wherein the phospholipid is a phosphatidylcholine or a phosphatidylserine.

3. The preventing or ameliorating agent according to claim 1, wherein the lysophospholipid is a lysophosphatidylcholine or a lysophosphatidylserine.

4. The preventing or ameliorating agent according to claim 1, wherein the agent is used for inhibiting skeletal muscle mass loss.

5. The preventing or ameliorating agent according to claim 1, wherein the agent is used for inhibiting lipoatrophy.

6. The preventing or ameliorating agent according to claim 1, wherein the agent is used for inhibiting expression of a proinflammatory cytokine.

7. The preventing or ameliorating agent according to claim 1, wherein the cachexia is induced by a disease, wherein the disease that induces the cachexia is a chronic disease associated with inflammation.

8. The preventing or ameliorating agent according to claim 7, wherein the chronic disease associated with inflammation is a disease selected from the group consisting of cancer, chronic heart failure, chronic obstructive lung disease, chronic renal failure, chronic liver diseases, chronic infections, sepsis, autoimmune diseases, and diabetes mellitus.

9. A method for screening for a substance for ameliorating cachexia, comprising selecting a test substance that promotes myotube formation as compared to that without addition of the test substance when culture is performed in an in vitro differentiation induction system of a stem cell or a progenitor cell into a skeletal muscle cell using a differentiation induction medium containing a culture supernatant of a cancer cell with addition of the test substance.

10. A method for screening for a substance for ameliorating cachexia, comprising selecting a test substance that promotes adipocyte differentiation as compared to that without addition of the test substance when culture is performed in an in vitro differentiation induction system of a stem cell or a progenitor cell into an adipocyte using a differentiation induction medium containing a culture supernatant of a cancer cell with addition of the test substance.
